(19) **Europäisches Patentamt**

**European Patent Office**

**Office européen des brevets**

(11) **EP 1 077 424 A1**

(12) **EUROPEAN PATENT APPLICATION**

(43) Date of publication:
**21.02.2001 Bulletin 2001/08**

(51) Int. Cl.⁷: **G06F 19/00**

(21) Application number: **00116053.0**

(22) Date of filing: **26.07.2000**

(84) Designated Contracting States:
**AT BE CH CY DE DK ES FI FR GB GR IE IT LI LU MC NL PT SE**
Designated Extension States:
**AL LT LV MK RO SI**

(30) Priority: **04.08.1999 EP 99115398**

(71) Applicant: **Flegel, Willy A., Dr.**
**64807 Dieburg (DE)**

(72) Inventors:
• **Flegel, Willy.**
**64807 Dieburg (DE)**
• **Wagner, Franz F.**
**89075 Ulm (DE)**

(74) Representative:
**VOSSIUS & PARTNER**
**Siebertstrasse 4**
**81675 München (DE)**

(54) **Method and apparatus for calculating a donor's likelihood of donating within a preselected time interval**

(57) The present invention provides a method and an apparatus for calculating a donor's likelihood of donating within a preselected time interval. The donation history of a donor is analyzed and a probability probability $p(D_{ts-te})$ of donation within said preselected time interval is calculated based on the result of the analysis of the donor's donation history.

EP 1 077 424 A1

**Description**

The present invention relates to a method and an apparatus for calculating a donor's likelihood of donating within a preselected time interval. Moreover, the present invention relates to a computer program comprising program code means for performing said method, to a computer program product, and to a method for establishing a data base comprising data representing a donor's donation history.

Many major blood donation services run blood drives at temporary countryside locations. Donors usually give blood at their village location or closeby, where blood drives are offered, e.g., two to four times a year. The usual minimum interval between blood drives is 82 days.

The procurement of some advanced blood components, like quarantined plasma units, depends critically on re-testing the donor within a fixed time frame. For health care systems, like in Germany, with mandatory retesting of donors before plasma release, the reliable identification of donors that are more likely to return in time has an immense practical implication, because their blood components could be preferably selected for quarantine purposes.

Advanced blood components, like quarantined plasma and cryopreserved red cell units, can be stored for months before being transfused. To further enhance the virus safety of such blood components, a mandatory re-testing of the donors for infectious disease markers has been introduced in the mid 1990s into transfusion practice of several countries, like Germany. Since then the supply of these blood components depended critically on the cooperation of the donors within a rather tight time frame after their index donation. Because less than half of all whole blood donations are required for sufficient supply with quarantine plasma units, a selection of donations suitable for quarantine became feasible. Thus, reliable identification of donors who can be expected to return within the relevant time frame would have an immense practical implication for health care systems with quarantine requirements. Plasma that does not meet quarantine criteria may only be used for plasma fractionation purposes.

Recently, Whyte (see Whyte G. Quantitating donor behaviour to model the effect of changes in donor management on sufficiency in the blood service. Vox Sang 1999; 76:209-15) analysed about 560,000 donation intervals and found the aggregate donor behaviour to be very predictable. With a simple regression analysis the attendance of repeat-donors and of first-time-donors was shown to be related.

James RC and Matthews DE (The donation cycle: a framework for the measurement and analysis of blood donor return behaviour. Vox Sang 1993; 64:37-42) developed a model based on a "donation cycle" by analysing about 180,000 blood group O donors and 610,000 donation attempts. To predict the time that will elapse since the previous donation attempt, they applied a Cox regression model (Cox DR, Oakes D. *Analysis of Survival Data*. London: Chapman and Hall, 1984) as used in standard time-to-outcome methods, which yield survival curves and relative risk estimates. They showed some influence of sex, Rhesus blood group, and rare donor status on donor return. They also analysed the donor history for up to four previous donation cycles and concluded that the elapsed time since a previous index donation affected the likelihood of a subsequent donation significantly.

It is the object of the present invention to provide a method and an apparatus for calculating a donor's likelihood of donating within a preselected time interval. This object is achieved with the features of the claims.

According to the invention, the following abbreviations are used:

$p(D)$ = probability of donation;

$p(D_{ts-te})$ = probability of donation within a preselected time interval $[t_s; t_e]$.

According to the invention, a donor's likelihood of donating within a preselected time interval is calculated by analyzing the donation history of a donor and calculating a probability probability $p(D_{ts-te})$ of donation within said preselected time interval based on the result of the analysis of the donor's donation history.

According to a second aspect of the present invention, a computer program comprising program code means for performing all the steps of the method according to the invention when said program is run on a computer is provided.

According to a third aspect of the present invention, a computer program product is provided, comprising program code means stored on a computer readable medium for performing the method of the invention when said program product is run on a computer.

According to a fourth aspect of the present invention, there is provided a method for establishing a data base comprising data representing a donor's donation history using for each donor a donor score, wherein at each donation a predetermined amount is credited to said donor score.

According to a fifth aspect of the present invention, there is provided a method for improving the rate of release after quarantine storage of human blood or blood components by using the available information for blood donation history or for other donor demographic factors or a combination thereof.

According to a sixth aspect, the present invention concerns the use of a method according to the present invention or an apparatus according to the present invention or a computer program or a computer program product according to the present invention for storing of blood, blood components, plasma derivatives or plasma preparations.

According to a seventh aspect, the present invention provides the use of a method or an apparatus or a computer program or a computer program product according to the present invention for quarantine storage of blood, blood components, plasma derivatives or plasma preparations.

The present invention provides a method and an apparatus for calculating a probability of donation, $p(D_{ts-te})$, within a given time frame ($t_s$-$t_e$). In difference to known approaches, the present invention obviates the need to calculate time intervals between donations and their variation. According to the invention, the utility of a donor's donation history is analyzed to calculate a "probability of donation", p(D), within a preselected time frame. The present invention allows to apply a simple logistic regression model, e.g. as described in Hosmer DW, Lemeshow S. *Applied Logistic Regression*. New York: John Wiley & Sons, 1989, and obviates the need for the more complex Cox regression required in the prior art.

Donor score

According to the invention, a donor score is defined in accordance with following formula 1.0 and, based on the donor's donation record, is preferably determined for any donor at, e.g., a blood donation service.

According to the invention, if a donor donated blood at the latest blood drive at his/her location before a current donation, i. e. at the most recent previous opportunity to donate blood, a "1" is added to a "credit". If the donor donates at the next most recent occasion, an „0.5" is credited. If the donor donates at the k-th previous occasion, another „1/k" is credited. In the present invention, „1/k" is defined as a weighting factor. This "credit" is compounded to a "score" applying

$$score = \sqrt{1 + \sum_{k=1}^{n} I\frac{1}{k}} \qquad (1.0)$$

$$I = \begin{cases} 1, & \text{if } k\text{ - th previous donation } = \text{ yes} \\ 0, & \text{if } k\text{ - th previous donation } = \text{ no} \end{cases}$$

with n representing the total number of possible donation attempts at the donor's location. Hence, for all first-time-donors the score is equal to 1, since no previous donation attempts were made, i.e., I=0. The square root transformation is done to achieve a good fit of the logistic link function in the regression analysis. The score gives more weight to recent donations and less weight to older donations.

For example, a first-time-donor has a score of $1 = \sqrt{1+0+0+...}$; for a donor who donates five times at all five most recent available occasions, the score is $1.51 = \sqrt{1+1/2+1/3+1/4+1/5+0+...}$; and for a donor with two donations at the current and at the 5-th most recent occasion available, the score is $1.10 = \sqrt{1+0/2+0/3+0/4+1/5+0+...}$ .

Logistic regression

According to the invention, in a logistic regression model the probability of donation, p(D), for a donor is modeled applying two explanatory variables, a score (see equation 1.0) for repeat-donors and a dichotomous variable, $donor_{FT}$, indicating a first-time-donors status. This model is formally represented by:

$$p(D) = Prob(donation|score, donor_{FT}) \qquad (1.1)$$

The probability of donation and the explanatory variables are linked by the logit function that is used to model the donor's behaviour and predict his/his p(D):

$$\log(p(D)/(1 - p(D))) = int + scf \cdot score + ftd \cdot donor_{FT} \qquad (1.2)$$

$$donor_{FT} = \begin{cases} 1, \text{ if first - time - donor } = \text{ yes} \\ 0, \text{ if first - time - donor } = \text{ no} \end{cases}$$

wherein int (intercept), scf (score factor) and ftd (first-time-donor) denote regression coefficients. The three regression coefficients, int, scf and ftd, are preferably determined by modeling with actual donation data sets (see below). The following ranges are preferred: int=[-3.88;-2.50]; scf=[2.30;3.29]; ftd=[0.05;0.60]. More preferably, int=-3.8410 or -3.7305; scf=2.4334 or 2.3456; ftd=0.5718 or 0.5470. By transforming this regression equation, the direct calculation of the probability of donation, p(D), is possible. For ease of use, the equations are shown separately for repeat- (1.3) and first-time-donors (1.4), but are preferably compounded in one formula (1.5) by using the dichotomous variable $donor_{FT}$.

The following logistic regression model

$$p(D_{ts - te}) = \frac{e^{int + scf \cdot score}}{1 + e^{int + scf \cdot score}} \qquad (1.3)$$

is used for repeat-donors. $t_s$ denotes time of start and $t_e$ time of end of the donation interval, D.

The value of the score obtained in equation 1.0 is preferably used for calculating the p(D) for repeat-donors by applying equation 1.3 with the appropriate coefficients for the desired prediction interval (see below).

First-time-donors are dealt with separately to improve the fit of the logistic regression model. The following logistic regression model

$$p(D_{ts - te}) = \frac{e^{int + scf + ftd}}{1 + e^{int + scf + ftd}} \qquad (1.4)$$

is used for first-time-donors.

Preferably, the formulae 1.3 and 1.4 are combined:

$$p(D_{ts - te}) = \frac{e^{int + scf \cdot score + ftd \cdot donorFT}}{1 + e^{int + scf \cdot score + ftd \cdot donorFT}} \qquad (1.5)$$

$$donor_{FT} = \begin{cases} 1, \text{ if first - time - donor } = \text{ yes} \\ 0, \text{ if first - time - donor } = \text{ no} \end{cases}$$

It should be noted, that the probability of donating within a certain time frame after an index donation is practically equal to the probability of retesting, because retesting among the donors occurs almost exclusively during the subsequent donation attempts.

Fig. 1    shows the ROC curve of the logistic regression model for $p(D_{170-275}$ days);
Fig. 2    shows the rate of quarantine success among all blood donations; and
Fig. 3    shows the relationship of plasma units put in quarantine storage and quarantine plasma units released.

Since 2 May 1985, a central electronic database for blood donation has been maintained at the DRK-Blutspendedienst Baden-Württemberg, Germany (see Wagner FF, Kasulke D, Kerowgan M, Flegel WA. Frequencies of the blood groups ABO, Rhesus, D category VI, Kell, and of clinically relevant high-frequency antigens in South-Western Germany. Infusionsther Transfusionsmed 1995; 22:285-90) comprising all donation related information including location and laboratory results. On 5 November 1998, records for 763,401 donors and 4,915,777 donations at 1,150 different locations were available. These data sets represented more than 7% of all residents in the *Land* (state of) Baden-Württemberg and more than 80% of all donations in that area.

These data are used for verification of the present invention. All donor data were reviewed and 49,219 donors (6,45%) were excluded from evaluation for reasons like inconsistent data or positive infectious disease markers. Donations at the three permanent blood donation sites of the blood donation service in the cities of Ulm, Mannheim and Baden-Baden were also excluded. Because of this measure, the examined data represented truthfully the donation patterns at temporary donation sites.

Since 1 January 1995, a six month quarantine before plasma units were released was observed, a procedure made mandatory on 1 July 1995 by the Germany regulatory authority, the Paul-Ehrlich-Institut, Langen. Starting in mid 1994, all donors were informed about the quarantine requirements and asked to return for re-testing purposes. The donors were notified by postcards once a blood drive was offered at the location of their most recent donation.

For verification of the present invention, descriptive donor statistics were performed by standard methods. The influence of various demographic characteristics on donor return within 170 to 275 days after an index donation was calculated in an univariate analysis. $R^2$, the coefficient of determination, was calculated as described by Nagelkerke (Nagelkerke NJD. A note on a general definiton of the coefficient of determination. Biometrika 1991; 78:691-2).

To determine relevant demographic factors for returning to a subsequent blood drive and to verify the present method for predicting the probability of donation within a preselected time frame the subset of 210,786 donors who donated blood between 1 October 1995 and 30 September 1996 was evaluated. The donors were assigned at random into three groups. With the first group a logistic regression model was constructed, which was validated with the second group as an independent sample. Because this model was considered satisfactory, a further modeling step was not required, for which the third group had been spared as another independent validation sample.

Using equation 1.0 above, the donor scores for index donations between 1 October 1996 and 30 September 1997 range from 1 to 2.327 (mean $\pm$ SD: 1.424 $\pm$ 0.313; median 1.404, 25%-quartile: 1.157, and 75%-quartile: 1.651). The maximum number of donations among the donors is 150; the 60 most recent donations were compounded in the score by applying formula 1.0.

With 69,975 donors of the first set, the modeling of a logistic regression was performed in accordance with the present invention to predict the likelihood of a subsequent donation within 170 days to 275 days: $p(D_{170-275 \text{ days}})$. For a $p(D_{170-275 \text{ days}})$, the regression coefficients were estimated for first-time-donors (ftd = 0.5718) and repeat-donors (int = - 3.8410 and scf = 2.4334). The $R^2$ of Nagelkerke representing a parameter for the fit of the present method was 0.13 and the area under the ROC curve (AUC) representing a parameter for the predictive value (see Hosmer) was 0.68.

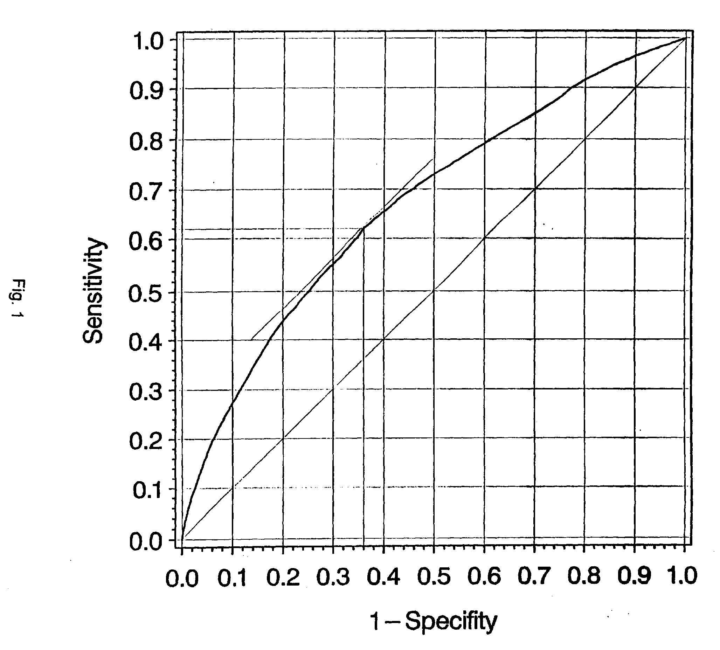

In an ROC curve analysis (see Fig. 1) the sensitivity (true-positive rate) is plotted against 1-specificity (false-positive rate). The area under the ROC curve (AUC) may vary between 0.5, which indicates chance level of prediction, and 1.0, which indicates completely accurate prediction. In Fig. 1, the curve particularly represents unbiased estimates for the sensitivity (true-positive rate) and 1 - specificity (false-positive rate) of the logistic regression model predicting the probability of donation, $p(D_{ts-te})$, during a given time frame ($t_s$-$t_e$) with $t_s$ = 170 days and $t_e$ = 275 days. For example, the present method can attain about 62% sensitivity with 64% specificity. The calculated area under the curve (AUC) is about 0.66. The curve is constructed with the data of an independent, second set of 69,948 donors by using the estimated regression coefficients that were derived from the data of the first set of 69,975 donors. The ROC curves obtained with the regression coefficients of Table 3 (see below) were almost identical (not shown).

Other donor demographic factors, like sex, age, blood group and size of donor community, have some predictive value when used alone (see Table 2 below), and these other demographic factors are preferably used in combination with the donor score.

The logistic regression model thus obtained was evaluated with 69.948 donors of the second set. As the results of the modeling was considered sufficient (see section Results below), there was no need to utilize the remaining, third set of donor data for evaluation purposes. It should be noted that there was at least one opportunity for the return visit for each index donation within the desired interval.

Blood components, like plasma units, that require quarantine storage were sorted according to their donors' $p(D_{ts-te})$ for one or more preselected time intervals, $t_s$-$t_e$. According to the known demand for quarantined units, a suitable cut-off value for $p(D_{ts-te})$ was determined for which the expected quarantine unit release equalled or exceeded the demand. The outcome was studied, if blood components of donors with $p(D_{ts-te})$ values equalling or exceeding the cut-off were put into quarantine storage to increase the success rate over the current procedure.

As an example, often the number of plasma units that can be stored will be determined by the given storage capacity (size of —40°C room). In this case, a $p(D_{cut \text{ off}})$ can be determined for which the number of donations with p(D) values higher than the cut off equals the number of units that can be stored for the average quarantine period. Only those donations with p(D) values higher than $p(D_{cut \text{ off}})$ will be deposited in quaratine storage. In such a way, the given storage capacity can be used in an optimal way.

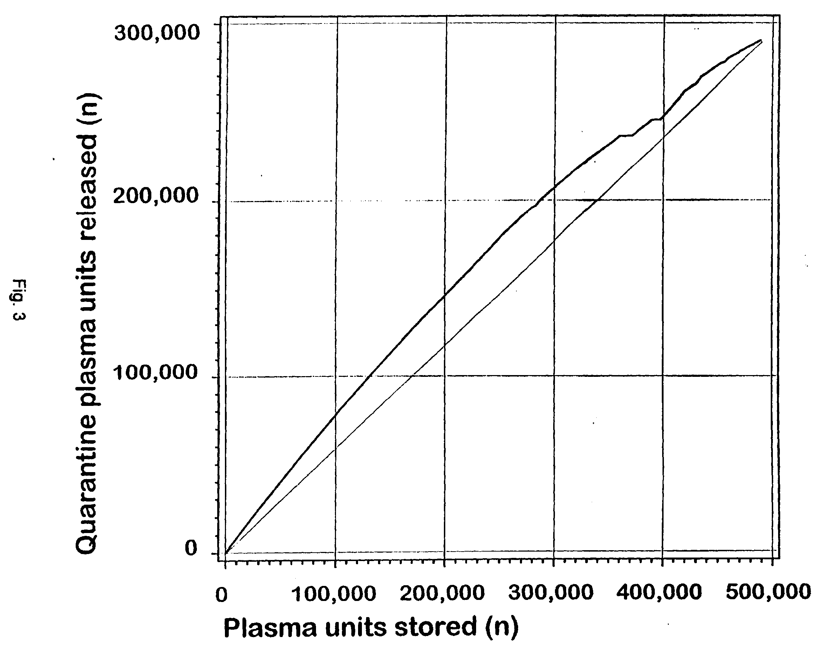

If a blood service is planning to establish storage capacity, the required size of a -40°C room will be determined by the number of plasma units that need to be released after the quarantine storage to meet the demand for patient care. The relationship between units stored and units released (see Fig. 3) are most useful for this purpose. The relationship with considering p(D) values for quarantine storage (Fig. 3, bold line) compares favourably with the standard approach neglecting p(D) values (Fig. 3, fine line). In Fig. 3, the figure relates the number of stored units with the maximum number of units released after quarantine, if storage would have been done according to the $p(D_{170-275\ days})$ in 1997. The average success rate for all 489,153 donations was 59.3%. The success rate of the present quarantine procedure (indicated by the bold line) is considerably higher than this average (indicated by the fine line) for a wide range of quarantine plasma units released. Please note the different scales for the x- and the y-axes.

All computations were done with the SAS package, release 6.12 (SAS Institute, Carey NC) for DEC OSF1 on an Alpha Server 1000A 5/333.

Results

An electronic database comprising 714.182 donors with 4,400,965 donation attempts was utilized in the verification of the present invention. The full dataset was evaluated by descriptive and univariate methods (see Tables 1 and 2 below). The present invention provides a donor score and a statistical model for the likelihood of donor return based on the donor score.

Various donor demographic factors were obtained for all donors and donation attempts and analysed for three equal periods spanning 14 years (see Table 1 below). An increase of the mean age occurred for repeat-donors by about one year and was even more apparent for one-time-donors. The fraction of women among one-time-donors increased to almost 50% in the most recent period. There may be a trend in the distribution of donors among villages, towns and major cities, as the large fraction of village dwellers among one-time donation attempts has even increased compared to the 1980s.

The effect of donor demographic factors on the rate of attending a subsequent donation within a preselected time interval (Table 2) was analysed. The donor status was the single most informative parameter predicting successful retesting. Age was the second most useful predictor. First-time-donors of age 18 were successful in returning, whereas in particular female donors in their twenties, possibly caused by pregnancies and nursing, were less reliable in this respect. The donor's reliability equalled that of 18-year-old first-time-donors at about age 30 and was further and steadily increasing with age until the early 60s.

Male donors returned more often than female (Table 2). Donors from major cities and towns were found to be less reliable than those from villages. It has previously been demonstrated by statistical analysis an excess of Rh neg. donors, in particular among female donors (data not shown). It has now been realized though, that Rh neg. donors were somewhat less successful in re-testing, which was likely caused by the observed lower return rate of female donors in general (Table 2). Likewise, the proportion of donations with blood group AB was about average, despite the high demand for plasma units of this blood group.

According to the invention, the donation record of a given donor indicates his/her likelihood of attempting another donation within a preselected time interval. The donor score is according to the invention determined for any donor of a blood donation service and is depended on the donors donation record only. This donor score is unique for any given index donation of a donor and is preferably be calculated for the donor's latest donation.

The donor scores mentioned above correlate with a $p(D_{170-275\ days})$ of 22% (for a score = 1) and 86% (for a score = 2.327) applicable to any index donation during the one year period in 1996/97. All first-time-donors have a $p(D_{170-275\ days})$ of 33%.

The logistic regression model thus obtained was evaluated with 69,948 donors of the second set. For this independent second set and a $p(D_{170-275\ days})$, very similar coefficients were obtained: ftd = 0.5470, int = -3.7305, scf = 2.3456. The predictive value of the present method was confirmed with this independent set of donors, because the values for $R^2$ = 0.12 and AUC = 0.67 were almost identical to those values for the first set of donors. An ROC curve for the second donor set is shown in Fig. 1.

The present method was applied to two different index donation intervals and two different time frames. As Table 3 shows, the coefficients of the model, $R^2$ that indicates the fit of the model and the area under the curve (AUC) that represents the predictive value do hardly change. This fact gives further evidence for the robustness of the present method.

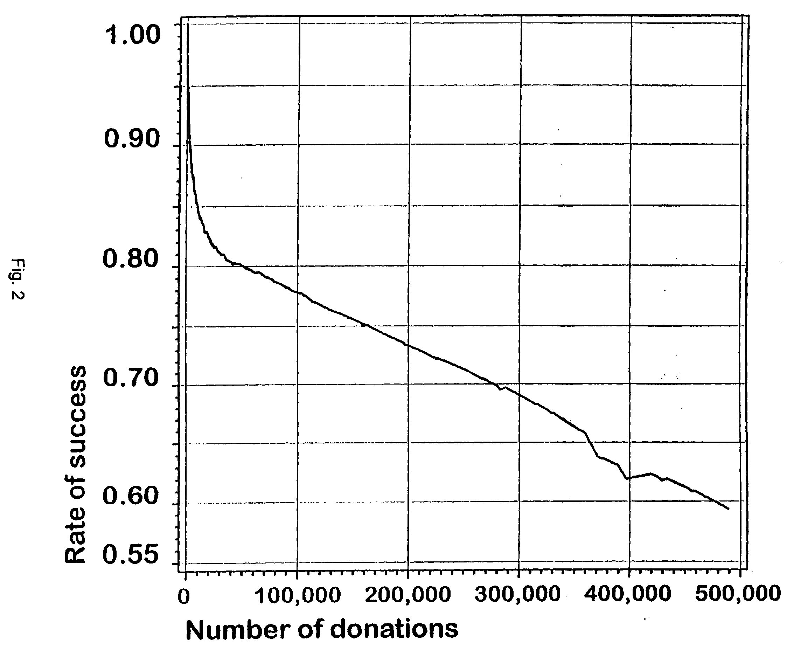

To demonstrate the utility of the calculated $p(D_{170-275\ days})$ for predicting the actual rate of returning, all donations in 1997 are sorted according to their calculated p(D), and the cumulative number of donations is plotted against the actual rate of returning within the time interval of 170 to 275 days (Fig. 2). This number increases, when the $p(D_{170-275\ days})$ is decreasing. On the ordinate, the actual rate of success is shown. Fig. 2 shows a smooth, almost monotonous curve demonstrating a good fit of the present method and an excellent prediction. Some very small subgroups were not optimally modeled and caused an increasing rate of success whilst the p(D) decreased. One major deviation is shown around 400,000 donations and is caused by first-time-donors, who contributed 59,388 (12.1%) of all donations. The donors returned within 170 to 275 days for 59.3% of all index donations. This percentage represents the average success rate for blood components quarantined at the tested blod service in 1997.

The overall, cumulated success rate averaged 59.3%, which was much higher than the mean donor return rate of 42.11% reported in Table 2. This virtual discrepancy between the success rate calculated for the cumulated donations and the donor return rate is explained, because reliable donors donate more often than less reliable donors.

The median $p(D_{170-275 \text{ days}})$ of all donors was 47% (range 22% to 86%, 25%-quartile: 33%, and 75%-quartile: 61%). The first-time-donors' success rate of 33% is much below that median. Hence, the overall success rate increases by 2.5 percentage points to 61.8%, if all first-time-donation are excluded from quarantine purposes. However, the first-time-donors have a better rate of success than some repeat-donors with a $p(D_{170-275 \text{ days}})$ as low as 22%. The application of the present method is hence far superior to the mere exclusion of plasma units of first-time-donors from quarantine storage.

Because plasma units expire after one year storage, the time interval of $t_s$=170 days to $t_e$=275 days is used in the present method. Preferably, $t_s$ is not shortened because the safety margin for the window period should not be jeopardized. As plasma storage for two years and longer may become standard, the effect of extending the time intervals by varying $t_e$ was tested (Table 4). Of course, more donors are successfully donating within the extented time frames and an extension of $t_e$ to 1.5 years would much improve the return rate. However, extenting $t_e$ beyond two years has a very marginal effect. The relative contribution of first time donors is much diminished beyond an interval of two years. The regression coefficients, first-time-donor (ftd), intercept (int) and score factor (scf), can be used to estimate $p(D)$ for a given time interval $t_s$-$t_e$ or to interpolate the coefficients for varying $t_e$. The present method is permissive for varying the time frames, because $R^2$ and AUC increased somewhat, indicating an even better prediction for longer time intervals.

If the plasma component of all donations in 1997 would have been stored, 288,600 units representing about 59% of all donations could have been released for transfusion in compliance with the minimum quarantine period of 170 days (Figure 3). The critical value of $p(D)$ useful as cut-off for plasma unit storage depends on the total number of stored units. As less than half of all donations were actually stored for quarantine purposes, the success rate of a quarantine procedure could be considerably enhanced above the current 59% average by selecting donations with a high $p(D)$ value attached.

For the supply of the *Land* (state of) Baden-Württemberg with 10 million inhabitants, the tested blood service produced about 100,000 quarantine plasma units per year. With the current success rate of about 59%, 175,000 units were needed to be put in storage. If the decision for storing any given plasma unit would have been based on its donor's $p(D)$ value, about 130,000 units could have retrieved without incurring any additional costs (Fig. 3). Vice versa, to achieve the release of 100,000 units it would have been necessary to keep 130,000 unit in storage rather than the 175,000 units actually stored per year (Fig. 3). If the storage costs were linear to the number of units stored, the application of the present method would have saved about 20% of the storage expenses.

The present method for predicting the donor's likelihood of donating within a preselected time interval could increase the available quarantine plasma units by about 30,000 units per year without incurring additional costs or reduce the quarantine storage expenses by about 20% without limiting the current supply. These figures, if widely applicable, may result in a welcome relief to the stringent cost containment efforts of the health care systems in Germany and elsewhere.

Previously donor demographic factors like age, age at first donation, sex, location of the blood drive and the donation history are preferably used for predicting the subsequent donation behavior. However, the donation history is most useful for predicting the probability of donation, $p(D)$. According to the invention, because no attempt is made to predict the exact number of days elapsing until the next donation, there is no need to rely on applying the Cox regression. Rather the present invention adopts the approach of a logistic regression model with a dichotomous variable, successful attending or not attending an offered blood drive at a particular location. The present invention is applicable to other services with similar donor characteristics at temporary donor sites, and it is also possible to determine the donor score for blood centers with the continuous option for donations rather than with blood drives at temporary locations.

As first-time-donors were rightfully perceived less likely to return than the average repeat-donor, their units were often not stored for quarantine purposes. The application of the present invention shows that this decision could improve the overall success rate by about 2.5 percentage points. Some repeat-donors who are often found among those with small numbers of previous donations were even less likely to return than first-time-donors. A rational decision tree for plasma quarantine would relegate donations of this subset of repeat-donors first, followed by all donations of first-time-donors. Then, donations of another subset of the remaining repeat-donors whose $p(D)$ did not exceed a certain threshold could be excluded from storage. The required $p(D)$ thresholds can easily be determined with considerable precision by the present invention.

Preferably, trends in certain demographic donor factors are utilized for $p(D)$ calculation. For example, the likelihood of first-time-donors to become repeat-donors was related to the age at first attempt; a notable exception were first-time-donors of 18 years, many of whom appeared to have "waited" for being eligible and were prone to develop to unusually reliable repeat-donors (data not shown). Likewise, the important effect of short-term, temporary deferral is preferably introduced as a parameter. If these parameters are compounded into the present method, the p(D) may be improved by a fraction of these parameters' isolated effects. The calculated score may be less than optimal for an individual donor, if certain events occurred, like change of address or pregnancies; in the aggregate analysis (Figures 2 and 3), however, less than optimal scores were compounded with "better" scores and the application to the actual datasets confirmed the suitability of "donor score" even if some scores were - and ever will be - predicted less than optimal.

As more plasma units of blood group AB are needed compared to those of other blood groups, different thresholds are preferably defined reflecting the relative demand. Several of the rare red cell units, e. g. O CCDee KK, are in short supply but still plentiful enough that not nearly all units need to be stored frozen in liquid nitrogen. The p(D) is preferably used to define cut-off values for the management of blood donations from such "rare" donors.

The p(D) lends itself to reconstruct the aggregate behaviour of donors at particular times or locations. The „bottom up method" by summarizing the individual p(D) values for predicting the aggregate behaviour at particular times or donation localities is more useful than the common method of observing of the aggregate behaviour only. For example, the donation opportunities may be enhanced at those locations where the donor population was shown to be more eager to participate by the aggregate p(D) donor values of particular donation sites. The present method improves and facilitates the planning of blood drives and of an adequate blood supply.

For the current demand of about 100,000 quarantined plasma units per year the present method improves the rate of recovery to about 78% from the current average of 59%. The $R^2$ in Table 3 is about 0.12, which implies that about 12% of the "donor's motivation" to donate within the preselected time interval can be explained by the present method using the combination of the two parameter "donor score" and "donor status". This figure compares very favorably with the $R^2$ in Table 2 showing that the parameters age, sex, residence and blood groups covered less than 1% each. Preferably, further factors, like multiple donors among the peer group and the family members or psychologic attitude favoring donation are used.

The present invention provides a logistic regression model to calculate a probability of donation, $p(D_{ts-te})$, within a preselected time frame ($t_s-t_e$). The donation history is compounded in a score and used for determining $p(D_{ts-te})$. A logistic regression model is provided with score and donor status as parameters; different regression coefficients applied to first-time-donors (ftd) and to repeat-donors (intercept, int, and score factor, scf). The present invention allows to determine the probability of donation, $p(D_{ts-te})$, within a preselected time interval, like six to nine months after an index donation. The $P(D_{ts-te})$ is preferably calculated for any donor of blood services. The $p(D_{170-275\ days})$ ranged from about 22% to 86% for any index donation in 1996/97. First time donors had a $p(D_{170-275\ days})$ of 33% and were more likely to return within the time interval than certain subsets of repeat-donors that can be defined by the present method. There is provided a technical procedure to increase the rate of plasma unit release after quarantine storage. By applying the model to a current plasma quarantine programme about 30% more units could be retrieved, which would represent about 30,000 units per year, without incurring additional costs. General implications for blood collection, like planning blood drives, are improved. The whole demand of a health care system for single plasma units may be met by quarantine plasma and their cost-efficiency can be improved.

Table 1. Donors and changes in their demographic factors since 1985.

| Parameter and characteristic | May 1985 - Oct 1989 | | Nov 1989 - Apr 1994 | | May 1994 - Nov 1998 | |
|---|---|---|---|---|---|---|
| | one-time-donor * | repeat-donor * | one-time-donor | repeat-donor | one-time-donor | repeat-donor |
| *Age (years)* | | | | | | |
| mean ± SD | 32.0 ± 12.2 | 36.8 ± 12.4 | 33.5 ± 12.2 | 37.1 ± 12.4 | 34.7 ± 12.2 | 37.9 ± 12.2 |
| median (25%-, 75%-quartile) | 27.6 (21.6, 41.5) | 36.7 (25.1, 47.0) | 30.0 (23.4, 42.3) | 36.2 (25.9, 48.0) | 32.1 (24.6, 43.2) | 36.8 (27.7, 47.7) |
| *Sex* | | | | | | |
| female | 41.5% | 36.2% | 45.8% | 39.6% | 48.3% | 42.4% |
| male | 58.5% | 63.8% | 54.2% | 60.4% | 51.7% | 57.6% |
| *Residence* † | | | | | | |
| major cities | 6.3% | 4.3% | 5.3% | 3.8% | 5.4% | 3.4% |
| towns | 24.5% | 19.9% | 21.1% | 19.5% | 21.3% | 19.9% |
| villages & countryside | 69.2% | 75.8% | 73.6% | 76.7% | 73.2% | 76.7% |
| *Total* ‡ | | | | | | |
| number (n) | 119,172 | 258,646 | 120,293 | 291,886 | 124,885 | 301,188 |
| percentage of total number | 31.5% | 68.5% | 29.2% | 70.8% | 29.3% | 70.7% |
| fraction of population (15 to 65 years) § | 1.77% | 3.85% | 1.71% | 4.15% | 1.77% | 4.26% |

\* Within the indicated time interval. Many but not all of the one-time-donors were first-time-donors.

†According to 1990 postal code. Major cities: >100,000 inhabitants (Stuttgart; Freiburg, Heidelberg, Heilbronn, Karlsruhe, Konstanz, Mannheim, Tübingen, Ulm); towns (current or former "*Kreisstädte*"): 10,000 - 100,000; villages & countryside: <10,000.

‡A repeat-donor may be counted as one-time-donor, if only one donation occurred within a given time period. Many donors wer counted repeatedly in two or three time intervals, if they were repeat-donors.

§In 1989 the population 15 to 65 years old in the *Land* (state of) Baden-Württemberg was 6,718,260 (1993: 7,036,736, 1997: 7,066,940; Statistisches Landesamt Baden-Württemberg, Stuttgart).

**Table 2.** Donors in mid 1990s and exemplary demographic factors.

| Parameter and characteristic | donors (n) * | proportion (%) † | R² ‡ |
|---|---|---|---|
| | Number of donors who donated within 170 to 275 days and their proportion among all donors with the same characteristic | | |
| *Donor status* | | | 0.013 |
| first-time-donor | 9,277 | 30.3% | |
| repeat-donor | 79,495 | 44.1% | |
| *Age* | | | 0.008 |
| *Distribution among females* | | | |
| 18 - 19 years | 865 | 39.0% | |
| 20 - 31 years | 9,345 | 34.2% | |
| 32 - 41 years | 9,352 | 40.5% | |
| 42 - 51 years | 7,409 | 41.4% | |
| 52 - 63 years | 7,026 | 42.8% | |
| 64 - 65 years | 88 | 27.6% | |
| *Distribution among males* | | | |
| 18 - 19 years | 807 | 39.8% | |
| 20 - 31 years | 12,865 | 38.3% | |
| 32 - 41 years | 14,589 | 44.7% | |
| 42 - 51 years | 12,500 | 47.3% | |
| 52 - 63 years | 13,737 | 48.6% | |
| 64 - 65 years | 189 | 27.0% | |
| *Sex* | | | 0.004 |
| female | 34,085 | 39.0% | |
| male | 54,687 | 44.3% | |
| *Residence* | | | 0.0007 |
| major cities | 2,173 | 36.8% | |
| towns | 17,649 | 40.7% | |
| villages & countryside | 68,950 | 42.7% | |
| *Blood groups* | | | 0.00008 ** |

| | | |
|---|---|---|
| O Rh neg. | 7,825 | 41.5% |
| A Rh neg. | 8,039 | 42.0% |
| O Rh pos. | 28,944 | 42.3% |
| A Rh pos. | 30,237 | 42.2% |
| AB Rh pos./Rh neg. | 4,313 | 42.8% |
| B Rh pos./Rh neg. | 9,413 | 41.8% |
| *Total* | 88,772 | 42.1% |

*A total of 210,786 donors, who were offered a blood drive and including the 64- and 65-year-old donors, were analysed for being re-tested within 170 to 275 days after their first donation between 1 October 1995 and 30 September 1996. For the donor-related statistics presented in this table, only the first donation of donors during this time period was analysed.

†Proportion of "successful" donors among all donors with the specified characteristic.

‡$R^2$, the coefficient of determination, may be interpreted as the proportion of the explained "variation" of the outcome. For example, an $R^2 = 0.013$ implied that 1.3% of the "donor's motivation" to attend re-testing can be explained by the parameter "donor status".

**For brevity, the data for Rh pos./Rh neg. were combined in the blood groups AB and B. However, $R^2$ was calculated for the eight different groups rather than the six groups shown.

**Table 3.** Coefficients and statistical parameters of the logistic regression model for two different index donation intervals and two different time frames.

| Index donation interval and variable | $p(D_{170\text{-}275\ days})$ | | | | $p(D_{82\text{-}365\ days})$ | | | |
|---|---|---|---|---|---|---|---|---|
| | first time donor (ftd)* | intercept (int)* | score factor (scf)* | general description | first time donor (ftd) | intercept (int) | score factor (scf) | general description |
| *between 1 Oct 1995 and 30 Sept 1996* | | | | | | | | |
| parameter estimate † | 0.5629 | -3.7988 | 2.4024 | | 0.5253 | -3.4287 | 2.8871 | |
| standard error | 0.0163 | 0.0285 | 0.0189 | | 0.0146 | 0.0297 | 0.0209 | |
| $\chi^2$ | 1,198 | 17,725 | 16,183 | | 1,294 | 13,324 | 19,162 | |
| p value | 0.0001 | 0.0001 | 0.0001 | | 0.0001 | 0.0001 | 0.0001 | |
| odds ratio | 1.76 | NA ‡ | 11.0 | | 1.69 | NA | 17.9 | |
| 95% confidence interval | 1.70 - 1.81 | NA | 10.6 - 11.5 | | 1.64 - 1.74 | NA | 17.2 - 18.7 | |
| $R^2$; AUC § | | | | 0.12; 0.67 | | | | 0.15; 0.70 |
| eligible donors (n) ** | | | | 209,768 | | | | 233,634 |
| proportion of successful donors | | | | 42.19% | | | | 64.91% |
| *between 1 Oct 1996 and 30 Sept 1997* | | | | | | | | |
| parameter estimate | 0.5629 | -3.5762 | 2.3114 | | 0.5609 | -3.4466 | 2.9798 | |
| standard error | 0.0164 | 0.0290 | 0.0191 | | 0.0150 | 0.0313 | 0.0220 | |
| $\chi^2$ | 1,173 | 15,199 | 14,630 | | 1,394 | 12,109 | 18,413 | |
| p value | 0.0001 | 0.0001 | 0.0001 | | 0.0001 | 0.0001 | 0.0001 | |
| odds ratio | 1.76 | NA | 10.1 | | 1.75 | NA | 19.9 | |
| 95% confidence interval | 1.70 - 1.81 | NA | 9.7 - 10.5 | | 1.70 - 1.81 | NA | 18.9 - 20.6 | |
| $R^2$; AUC | | | | 0.12; 0.67 | | | | 0.15; 0.70 |
| eligible donors (n) | | | | 201,249 | | | | 228,336 |
| proportion of successful donors | | | | 45.04% | | | | 67.84% |

\* Degrees of freedom = 1.

† Estimates for the logistic regression coefficients.

‡ NA - not applicable.

§ $R^2$ represents a parameter for the fit of the present method and AUC represents a parameter for the predictive value of the method.

** Only those donors were evaluated who had been offered a blood drive at their preferred location during the indicated time frame, excluding all donors older than 63 years.

Table 4

| The effect of varying the length of the time interval ($t_s$-$t_e$) with $t_s$=170 days kept constant.* | | | | | | |
|---|---|---|---|---|---|---|
| | | Estimates for the logistic regression coefficients | | | | |
| end of time interval ($t_e$) | successful donors (%) | first time donor (ftd) | intercept (int) | score factor (scf) | $R^2$ † | AUC † |
| 275 days | 42.3 | 0.54 | - 3.88 | 2.49 | 0.12 | 0.67 |
| 1 year | 56.2 | 0.44 | - 3.48 | 2.66 | 0.13 | 0.68 |
| 1.5 years | 71.6 | 0.33 | - 3.15 | 2.99 | 0.14 | 0.70 |
| 2 years | 77.7 | 0.29 | - 3.11 | 3.24 | 0.15 | 0.71 |
| 2.5 years | 81.1 | 0.19 | - 2.87 | 3.25 | 0.14 | 0.72 |
| 3 years | 82.9 | 0.14 | - 2.77 | 3.29 | 0.14 | 0.72 |
| 3.5 years | 84.2 | 0.08 | - 2.61 | 3.26 | 0.14 | 0.72 |
| 4 years | 85.1 | 0.05 | - 2.50 | 3.24 | 0.14 | 0.72 |

\* A total of 187,435 donors were evaluated with at least one donation between 1 October 1993 and 30 September 1994.
† For $R^2$ and AUC see legend to Table 3.

## Claims

1. Method for calculating a donor's likelihood of donating within a preselected time intervall [$t_s$;$t_e$], comprising the steps:

   a) analyzing a donor's donation history; and
   b) calculating a probability $p(D_{ts\text{-}te})$ of donation within said preselected time interval based on the result of the analysis of the donor's donation history.

2. The method of claim 1, wherein step a) comprises the step of calculating a donor score based on a donor's donation record representing said donor's donation history.

3. The method of claim 2, further comprising the step of weighting subsequent donations with weighting factors.

4. The method of claim 3, wherein said weighting factors decrease from the first donation to the most recent donation.

5. The method of claim 2, 3 or 4, wherein the donor score is calculated using the equation

$$score = \sqrt{1 + \sum_{k=1}^{n} I \frac{1}{k}}$$

$$\text{with } I = \begin{cases} 1, & \text{if k - th previous donation} = \text{yes} \\ 0, & \text{if k - th previous donation} = \text{no} \end{cases}$$

and n representing the total number of possible donation attempts at locations available to the donor.

6. The method of any of claims 1 to 5, wherein in steb b) a logistic regression model is used for calculating said probability $p(D_{ts\text{-}te})$ of donation within said preselected time interval.

**7.** The method of any of claims 2 to 6, wherein said calculating step is further based on a dichotomous variable *donor*$_{FT}$ indicating a first-time-donor status, wherein

$$\text{donor}_{FT} = \begin{cases} 1, & \text{if first-time-donor} = \text{yes} \\ 0, & \text{if first-time-donor} = \text{no} \end{cases}.$$

**8.** The method of claim 6 or 7, wherein said probability $p(D_{ts-te})$ of donation within said preselected time interval is calculated using equation

$$p(D_{ts-te}) = \frac{e^{\text{int} + \text{scf} \cdot \text{score} + \text{ftd} \cdot \text{donorFT}}}{1 + e^{\text{int} + \text{scf} \cdot \text{score} + \text{ftd} \cdot \text{donorFT}}},$$

wherein int, scf, and ftd denote regression coefficients.

**9.** The method of claim 8, wherein int is in a range from -3.88 to -2.50, and preferably -3.8410 or -3.7305, scf is in range from 2.30 to 3.29, and preferably 2.4334 or 2.3456, and ftd is in the range from 0.05 to 0.6, and preferably 0.5718 or 0.5470.

**10.** Apparatus for calculating a donor's likelihood of donating within a preselected time intervall [$t_s$;$t_e$], comprising:

    means for analyzing a donor's donation history; and
    means for calculating a probability $p(D_{ts-te})$ of donation within said preselected time interval based on the result of the analysis of the donor's donation history.

**11.** The apparatus of claim 10, wherein said analyzing means comprises means for calculating a donor score based on a donor's donation record representing said donor's donation history.

**12.** The apparatus of claim 11, further comprising means for weighting subsequent donations with weighting factors.

**13.** The apparatus of claim 12, wherein said weighting factors decrease from the first donation to the most recent donation.

**14.** The apparatus of claim 11, 12 or 13, for performing the method of any of claims 5 to 9.

**15.** Method for improving the rate of release after quarantine storage of human blood or blood components by using the available information for blood donation history or for other donor demographic factors or a combination thereof.

**16.** A computer program comprising program code means for performing all the steps of anyone of the claims 1 to 9 when said program is run on a computer.

**17.** A computer program product comprising program code means stored on a computer readable medium for performing the method of anyone of the claims 1 to 9 when said program product is run on a computer.

**18.** Method for establishing a data base, preferably using the method of any of claims 1 to 9 or 15 or the apparatus of any of claims 10 to 14, said data base comprising data representing a donor's donation history using for each donor a donor score, wherein at each donation a predetermined amount is credited to said donor score.

**19.** Use of a method according to any of claims 1 to 9 or 15 or 18 or an apparatus according to any of claims 10 to 14 or a computer program according to claim 16 or a computer program product according to claim 17 for storing of blood, blood components, plasma derivatives or plasma preparations.

**20.** Use of a method according to any of claims 1 to 9 or 15 or 18 or an apparatus according to any of claims 10 to 14 or a computer program according to claim 16 or a computer program product according to claim 17 for quarantine storage of blood, blood components, plasma derivatives or plasma preparations.

Fig. 1

Fig. 2

Fig. 3

European Patent
Office

**EUROPEAN SEARCH REPORT**

Application Number

EP 00 11 6053

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X | BURNETT J.J.: "Distinguishing characteristics of blood donor segments defined in terms of donation frequency" JOURNAL OF HEALTH CARE MARK, vol. 6, no. 2, June 1986 (1986-06), pages 38-48, XP000965365 * page 38, line 1 - page 47, right-hand column, line 12 * | 1,2,10, 11,15-18 | G06F19/00 |
| D,X | JAMES R C ET AL: "THE DONATION CYCLE: A FRAMEWORK FOR THE MEASUREMENT AND ANALYSIS OFBLOOD DONOR RETURN BEHAVIOUR" VOX SANGUINIS,S. KARGER AG, BASEL,CH, vol. 64, no. 1, January 1993 (1993-01), pages 37-42, XP000965323 ISSN: 0042-9007 * abstract * | 1,2,10, 11 | |
| Y | * page 37, left-hand column, line 1 - page 42, right-hand column, line 14 * | 15 | |
| Y | SIRELSON V ET AL: "A computer planning model for blood platelet production and distribution" COMPUTER METHODS AND PROGRAMS IN BIOMEDICINE, AUG. 1991, NETHERLANDS, vol. 35, no. 4, pages 279-291, XP000961489 ISSN: 0169-2607 * page 279, left-hand column, line 1 - page 281, right-hand column, line 21 * | 15 | TECHNICAL FIELDS SEARCHED (Int.Cl.7) G06F |

-/--

The present search report has been drawn up for all claims

| Place of search | Date of completion of the search | Examiner |
|---|---|---|
| THE HAGUE | 20 December 2000 | Schenkels, P |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

& : member of the same patent family, corresponding
  document

EPO FORM 1503 03.82 (P04C01)

European Patent
Office

**EUROPEAN SEARCH REPORT**

Application Number

EP 00 11 6053

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | VAQUE J: "Computer system for blood donors management" MEDICAL INFORMATICS EUROPE 78, CAMBRIDGE, UK, 4-8 SEPT. 1978, pages 775-781, XP000964655 1978, Berlin, West Germany, Springer-Verlag, West Germany ISBN: 3-540-08916-0 * page 776, line 4 - page 779, line 28 * | 1,2,10, 11,15 | |
| A | PAGE B: "A review of computer systems in blood banks and discussion of the applicability of mathematical decision methods" METHODS OF INFORMATION IN MEDICINE, APRIL 1980, WEST GERMANY, vol. 19, no. 2, pages 75-82, XP000617876 ISSN: 0026-1270 * page 75, left-hand column, line 1 - page 77, left-hand column, line 12 * | 1,10,15 | |
| | | | TECHNICAL FIELDS SEARCHED (Int.Cl.7) |

The present search report has been drawn up for all claims

| Place of search | Date of completion of the search | Examiner |
|---|---|---|
| THE HAGUE | 20 December 2000 | Schenkels, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)